(19) Europäisches Patentamt
European Patent Office
Office européen des brevets

(11) **EP 0 996 429 B1**

(12) **EUROPEAN PATENT SPECIFICATION**

(45) Date of publication and mention
of the grant of the patent:
**05.02.2003 Bulletin 2003/06**

(21) Application number: **98955458.9**

(22) Date of filing: **15.10.1998**

(51) Int Cl.[7]: **A61K 9/14**

(86) International application number:
**PCT/EP98/06544**

(87) International publication number:
**WO 99/021534 (06.05.1999 Gazette 1999/18)**

(54) **SOLID STATE SOLUTIONS AND DISPERSIONS OF POORLY WATER SOLUBLE DRUGS**

FESTE LÖSUNGEN UND DISPERSIONEN VON EINES SCHLECHT WASSERLÖSLICHEN
WIRKSTOFFES

SOLUTIONS A L'ETAT SOLIDE ET DISPERSIONS DE MEDICAMENTS TRES FAIBLEMENT
SOLUBLES DANS L'EAU .

(84) Designated Contracting States:
**AT BE CH DE DK ES FI FR GB GR IE IT LI LU NL
PT SE**

(30) Priority: **27.10.1997 US 63338 P**

(43) Date of publication of application:
**03.05.2000 Bulletin 2000/18**

(73) Proprietor: **MERCK PATENT GmbH
64293 Darmstadt (DE)**

(72) Inventors:
• **TALLAVAJHALA, Siva, Narayan
Dix Hills, NY 11746 (US)**
• **LIU, Xiuying
Cherry Hill, NY 08003 (US)**

(56) References cited:
**WO-A-96/21439          WO-A-98/43635
US-A- 5 487 887**

• **MAES, P. ET AL: "In vitro and in vivo behavior of
some liquid or semi-solid filled hard gelatin
capsules" BULL. TECH./GATTEFOSSE REP.
(1996), 89, 63-70 CODEN: BTGRDQ;ISSN:
0397-7617, XP002099025**
• **DORDUNOO, S. K. ET AL: "Preformulation
studies on solid dispersions containin
triamterene or temazepam in polyethylene
glycols or gelucire 44/14 for liquid filling of hard
gelatin capsules" DRUG DEV. IND. PHARM.
(1991), 17(12), 1685-713 CODEN: DDIPD8;ISSN:
0363-9045, XP002099026**
• **GINES, J. M. ET AL: "Elaboration and thermal
study of interactions between cinnarizine and
Gelucire 53/10 physical mixtures and solid
dispersions" INT. J. PHARM. (1995), 126(1,2),
287-91 CODEN: IJPHDE;ISSN: 0378-5173,
XP002099028**

## Description

### Field of the Invention

This invention relates to a composition especially useful as a pharmaceutical solutibility excipient, the method of producing same, and the pharmaceutical compositions obtained thereby. In particular, the invention has applicability to increasing the solubility of poorly soluble therapeutically active compounds.

### Summary of the Invention

The invention provides a pharmaceutical composition according to claim 1.

The invention further provides methods for making the above composition. In the first instance (a) and (b) are mixed to form an excipient mixture. In the second instance, to form a pharmaceutical composition, heating said polyglycolyzed glycerides and polyoxypropylene-polyoxyethylene block co-polymer sufficiently to melt the ingredients,

adding the therapeutic agent to the molten mixture of polyglycolyzed glycerides and polyoxypropylene-polyoxyethylene block co-polymer

maintaining the mixture at a sufficient temperature for a sufficient time to dissolve or disperse the pharmaceutical agent.

The method aspect of the invention further comprises adding one or more optional excipients, whereby:

(A) the solubility of the therapeutically-active compound in the polyglycolyzed glyceride:polyoxypropylene-polyoxyethylene block co-polymer mixture is increased, or

(B) the melting point of the non-drug components is set, whereby at least one melting point peak belonging to the non-drug components is present between 30-80°C in the final composition, when analyzed by thermal analytical techniques.

The method aspect of the invention further comprises

maintaining the resulting mixture in the molten form, with constant stirring to ensure homogenous distribution of the drug in the system, and then

subjecting the molten mixture to one or more of the following operations:

I) allowing the mixture to congeal to a solid mass, and then extruding the mixture through a hot melt extruder into a powder;

II) milling the mixture using equipment that would maintain the milling conditions at room temperature or below the melting point of the non-drug components of the system to enable milling the composition into a powder;

III) spray-congealing the mixture in a spray drier or fluidized bed drier to a powder;

IV) congealing the mixture onto one or more optional excipients in a spray drier, fluidized bed drier, rotor, high shear granulator, planetary mixer, blender, or any conventional food and pharmaceutical processing equipment;

V) formulating the above-mentioned powders in pharmaceutical tablets, capsules, powders for inhalation, suppositories, suspensions, and emulsions; or

VI) congealing said mixture onto a solid pharmaceutical tablet, capsule or granule.

It has been discovered that by utilizing a mixture of: (a) saturated or unsaturated polyglycolyzed glycerides, as exemplified by the commercial Gelucire compositions and (b) polyoxypropylene-polyoxyethylene block copolymers, as exemplified by the commercial Pluronic surfactants, it is possible to prepare solid state solutions or solid state dispersions containing poorly soluble therapeutically active compounds, which compositions, in turn, provide a high degree of solubility to the therapeutic agent.

Maes et al. describe the use of a mixture of Gelucire® 44/14 and poloxamer as excipients for pharmaceutical agents in liquid or semi-solid filled hard gelatin capsules (MAES, P. ET AL: 'In vitro and in vivo behavior of some liquid or semi-solid filled hard gelatin capsules' BULL. TECH./GATTEFOSSE REP. (1996), 89, 63-70). WO-A-96 21439 also discloses hard gelatine capsules filled with a mixture of Fenofibrate, Gelucire® 44/14 and Poloxamer 407 (example 1).

Gelucires® are polyglycolyzed glycerides prepared by the alcoholysis reaction of natural oils with polyoxyethylene glycols. They are mixtures of monoesters, diesters and/or triesters of glycerides of long chain $C_8$ to $C_{18}$ fatty acids, and in polyethylene glycol mono- and/or diesters of long chain fatty acids. These preparations have a wide range of melting points of from about 33°C to 64°C, as well as a wide range of hydrophilic/lipophilic balance values (HLB) from about I to about 14. The Gelucires® of particular interest in the present invention have an HLB of above 10.

**EP 0 996 429 B1**

The first number in the nomenclature of a Gelucire® denotes its melting point, whereas the second number provide the HLB value. The preferred Gelucires® of the present invention are grades 44/14 and 50/13.

The Pluronic® surfactants are block copolymers of polyoxyethylene and polyoxypropylene, generally having an average molecular weight from about 3,000 to about 15,000. The ethoxylated portion of the blocked copolymer generally constitutes from about 30 to about 80% by weight of the molecule. Particularly good results are achievable with Pluronic® F68, F108 and F127, but in any case, it is to be noted that the Pluronic® constituent should also have an HLB above 10, irrespective of the particular grade which is selected. For example, Pluronic® F108 has an average molecular weight of 14,600, a polyoxyethylene content of about 80 weight % and an HLB value in excess of 24, and Pluronic® F127 has an average molecular weight of 12,600, a polyoxyethylene content of about 70 weight % and an HLB value from 18 to 23.

The weight ratio of the combined saturated polyglycolyzed glycerides: polyoxypropylene-polyoxyethylene block co-polymer generally range between 0.10-99.9 to 99.9:0.10, with preferred ratios being 1:9, 2:8, 3:7, 4:6, 5:5, 6:4, 7:3, 8:2, and 9:1. These combinations especially the 5:5 ratio, yields a mixture having a melting point in the range of 44-70°C, preferably 50°C-70°C.

The composition of saturated polyglycolized glyceride and polyglyoxyproplyene-block copolymer is combined with a therapeutic agent, wherein said composition is present in the final composition, the latter including the therapeutic agent, in a range of about 0.10-99.9% by weight, with the preferred range being 5-75% by weight of the final composition. Poorly soluble agents, e.g., therapeutic agents which have an intrinsic water solubility of less than 10.0 g/l are particularly benefitted by the present invention. Examples of drugs in this category are drugs belonging to the dihydropyridine class of compounds (e.g., nifedepine, felodipine, nicardipine), omperazole, spironolactone, furosemide, terbutaline, riboflavine, gemfibrozil, indomethacin, ibuprofen, phenytoin, glyburide. In addition, any drug which has a water solubility of less than 10.0 g/l belonging to, for example, cardiovascular, cholesterol lowering, anti-hypertensive, antiepileptic, hormonal, hypoglycemic, antiviral, immunosuppressive, antihistaminic, nasal decongestant, antimicrobial, antiarrthrytic, analgesic, antimycobacterial, anticancer, diuretic, antifungal, antiparasitic, protein, peptide, CNS stimulants, CNS depressants, 5-HT inhibitors, anti-schizophrenia, anti-Alzheimer, antpsoriatic, steroidal, oligonucleotide, antiulcer, proton pump inhibitor, anti asthmatic, bronchodialators, thrombolytics, vitamin class of therapeutic agents, any combinations thereof may be used in this composition in order to form solid state solutions and dispersions.

The final composition optionally comprises the following further excipients at 5-95%, especially 10-70% by weight of the final composition. Examples of the further excipients include, but are not limited to ascorbyl palmitate, glycerol, glyceryl monooleate, glyceryl monosterate, glyceryl palmitosterate, triglycerides, diglycerides, monoglycerides, diesters of PEG, monoesters of PEG, polyethylene glycol, glycery polyoxyethlene fatty acid esters, glyceryl polyoxyethylene polyethylene glycol fatty acid esters and ethers, polyoxyethylene alkyl ethers, polyoxyethylene sorbitan fatty acid esters, polyoxyethylene sterates, polyvinyl alcohol, sorbitan fatty acid esters, polyoxyl sterates, polyethylene glycol hydroxysterate, polyoxyethylene alcohols, anionic, cationic, amphiphillic compounds, carbohydrates (lactose, maltodextrins, sucrose, starch, etc.), polyols (sorbitol, mannitol, xylitol, etc.), microcrystalline cellulose, vitamins (ascorbic acid, niacinamide, etc.), and inorganic compounds (calcium carbonate, dicalcium phosphate), polyoxyethylene castor oil derivatives, propylene carbonate, anionic emulsifying wax, white wax, yellow wax, hydrogenated vegetable oil, triacetin, triethyl citrate and other plasticizers (food and pharmaceutical grade), lecithin, phospholipids, soybean oil, sesame oil, cotton seed oil, sunflower oil, peanut oil, mineral oil, hydrogenated castor oil, water soluble and insoluble derivatives of cellulose (e.g. ethyl cellulose, methyl cellulose, HPMC, HPC, cellulose acetate phthalate, etc.), methacrylates and polymethacrylates (e.g., Eudragit®), canola oil, benzoic acid and its salts, methyl-, propyl- and butyl-para-amino benzoic acid (paraben) (preservatives), organic acids (e.g., fumaric, adipic, maleic, etc.), ethyl alcohol, saccharine, cyclamate sodium, and other artificial sweeteners, food and pharmaceutical flavoring agents, bioflavanoids (e.g., quercetin, isoquercetin), citrus bioflavanoids (e.g., naringin), citrus bioflavanoid complexes, and other agents that inhibit the enzyme cytochrome P450 4A4 (also called as CYP3A4), galactose oligosaccharides (example of a functional carbohydrate), lubricant (e.g. magnesium sterate), anti-caking agent (e.g., silicon dioxide, sodium aluminum silicate, magnesium trisilicate, talc, etc.), gums (locust bean gum, gum arabic, arabinogalactan, etc.); and any combination of said excipients.

To enhance the solubility of the therapeutically active agent, an aspect of this invention provides that the composition of the therapeutic reactive agent, the polyglycolyzed glyceride and the polyoxypropylene-polyoxyethylene block co-polymer is formed into a solid state solution or solid state dispersion.

A solid state solution is defined as a solution of the drug in a solid form. A solid state solution of a drug is characterized by the lack of a melting point peak at the melting point of the drug indicating the absence of the solid state of the drug. A solid state solution-dispersion is defined as a system in which part of the system may be in the solid solution form and part of it may be in the form of a finely dispersed solid form in the system. This solid state solution - dispersion is further defined as a system in which more than 1% of the total drug content can exist as a solid solution and more than 1% of the drug can exist as a solid dispersion with a particle size distribution such that 90% of the particles have a diameter less than 10 microns. The weight ratio between the solid state solubilized drug: dispersed drug may be in the range of 1.0-100:99-0. It is desirable that 30-100% of the drug exists as a solid state solution. The ratio of the amount of drug present in the form of a solid state solution to the amount present as a solid dispersion is easily ascertained by the use of techniques in thermal analysis such as Differential Scanning Calorimetry (DSC), Thermal Gravimetric Analysis (TGA), and Differential Scanning Microcalorimetry. The crystallinity of the drug is easily determined by X-ray diffraction. Furthermore, when determined by thermal analytical techniques it is desirable that the final composition have at least "one" distinct melting peak in the range of 30-80°C associated with the melting point of the non-drug components of the final composition.

To produce the final composition polyglycolyzed glycerides and polyoxypropylene-polyoxyethylene block co-polymer are heated sufficiently to form a melt of the ingredients, for example to at least about 20°C above the combined melting point. The therapeutic agent is added gradually to the molten mixture of polyglycolyzed glycerides and polyoxypropylene-polyoxyethylene block co-polymer. It is preferable to mill or micronize the drug to a particle size range such that the particle diameter of 90% of the particles is less than 75 microns. The mixture is maintained at a sufficient temperature, for example, at least about 20°C above the combined melting point of the polyglycolyzed glycerides: polyoxypropylene-polyoxyethylene block co-polymer mixture for a sufficient time to dissolve or disperse the pharmaceutical agent.

The optional excipients may be added to the above mentioned system to,

(A) Increase the solubility of the drug in the polyglycolyzed glycerides: polyoxypropylene-polyoxyethylene block co-polymer system.

(B) Set the melting point of the non-drug components, such that at least "one" melting point peak belonging to the non-drug components is present between 30-80°C in the final composition, when analyzed by thermal analytical techniques.

The resulting mixture is then maintained in the molten form, for example, at least 20°C above the combined melting point of the non-drug components, with constant stirring to ensure homogenous distribution of the drug in the system. Thereafter, the mixture may be subjected to one or more of the following operations:

I) Allowed to congeal to a solid mass, and then extruded through a hot melt extruder into a powder.

II) Milled using equipment that would maintain the milling conditions at room temperature or below the melting point of the non drug components of the system to enable milling the composition into a powder.

III) Spray congealed in a spray drier or fluidized bed drier to a powder.

IV) Congealed onto one or more of said excipients in a spray drier, fluidized bed drier, rotor, high shear granulator, planetary mixer, blender, or any conventional food and pharmaceutical processing equipment.

V) The above mentioned powders may then be used in the formulation of conventional, specialized, and novel pharmaceutical dosage forms such as tablets, capsules, powders for inhalation, suppositories, suspensions, and emulsions.

VI) Alternatively, the molten mixture can be congealed into or onto a solid pharmaceutical dosage form such as, for example, a tablet, capsule, and/or granule.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

In the foregoing and in the following examples, all temperatures are set forth uncorrected in degrees Celsius and unless otherwise indicated, all parts and percentages are by weight.

## EXAMPLES

### Example 1:

Three grams of Gelucire® of 50/13 and 3 grams of Pluronic® F68 are melted and two grams of nifedipine are dissolved therein. The resultant solution is added to 4 grams of sorbitol while stirring. The resultant solution is then cooled down and passed through a 20 mesh screen. The resultant particulate solids were solid solutions.

### Example 2:

In this example, felodipine is employed as the active pharmaceutical agent. Thus, 1.5 grams of Gelucire® 50/13 and 1.5 grams of Pluronic® F68 were melted together and 1 gram of felodipine was added thereto. This resultant solution was then introduced into 4 grams of Sorbitol P300® while stirring. After mixing, the solution was cooled and ultimately passed through a 20 mesh screen. The resultant particulate solids were in a solid state solution-dispersion system.

**Example 3:**

In this example, felodipine is employed as the active pharmaceutical agent. Thus, 1.5 grams of Gelucire® 50/13 and 1.5 grams of Pluronic® F68 were melted together and 1.5 grams of felodipine was added thereto. This resultant solution was then introduced into 4 grams of Sorbitol P300® while stirring. After mixing, the solution was cooled and ultimately passed through a 20 mesh screen. The resultant particulate solids were in a solid state solution system.

**Example 4**:

In this example, felodipine is employed as the active pharmaceutical agent. Thus, 1 gram of Gelucire® 50/13 and 1 gram of Pluronic® F68 were melted together and 1.5 grams of felodipine was admixed. This resultant solution was then added to 2.67 grams of Avecil®, a brand of microcrystalline cellulose. After mixing, the solution was cooled and ultimately passed through a 20 mesh screen. The resultant particulate solids were in a solid state solution-dispersion system.

For the purposes of increasing the melting point of the composition of Examples 2, 3 and 4, Pluronic® F127 can be substituted for Pluronic® F68.

To determine the improvement achieved by the present invention with respect to the solubility of pure felodipine, the formulations of Examples 3 and 4 were evaluated by the following technique.

The solubility characteristics of the compositions of Examples 3 and 4 as compared to pure felodipine were evaluated, as follows:

- 6.8 mg of felodipine and 34.8 mg of the composition of Example 3 and 34.2 Example 4 are respectively placed into 500 ml of a 40% PEG solution maintained at 37°C.

The solution was stirred with a paddle stirrer at 50 rpm. The absorbency was measured with a Hitachi spectrometer at 362 nm. The percent release is based on the standard curve: absorbents = 0.216 conc. (mg/900 ml)-0.00274, and the following results were obtained.

| Percent released (%) | | | |
|---|---|---|---|
| Time (h) | Pure felodipine | Example 3 | Example 4 |
| 0.5 | 9.2 | 62.4 | 65.6 |
| 1 | 21.1 | 76.4 | 78 |
| 2 | 49.5 | 84.4 | 84.6 |
| 3 | 66.8 | 85.7 | 86.3 |

From the above table, it is clear that the present invention provides a substantially enhanced solubility as compared to the pure drug.

**Example 5**:

For the production of tablets 121.5 mg of HPMC (hydropropylmethyl cellulose) are mixed with 21.1 gm of Sorbitol Instant P300®, 246.88 mg of Microcrystalline Cellulose and 59.62 mg of the composition of Example 3. 1 mg of magnesium stearate was then added to the above mixture with stirring. The resultant mixture was tableted in a Carver® press under a pressure of 2 tons.

**Example 6:**

Another tablet was produced from the composition of Example 3 by the method of Example 5 except in this case, the amount of Example 3 was 59.52 mg, Microcrystalline Cellulose 231.98 mg, Sorbitol Instant P300® 45 mg and HPMC 112.5 mg, with sodium stearyl fumarate (PRUV)® being substituted for magnesium stearate.

The resultant tablet has 10% sorbitol.

**Example 7**:

In this example, a 15% sorbitol tablet is produced in the same manner as the last example except that the amount of sorbitol is 67.5 mg and the amount of Microcrystalline Cellulose is 209.48 mg.

**Example 8:** Intrinsic Dissolution of Hydrosolve-Ibuprofen

a. Formulation:

| Excipient | Quantities (g) | Supplier |
|---|---|---|
| Ibuprofen | 2.5 | Albermarle |
| Pluronic® F68 | 2 | BASF |
| Gelucire® 50/13 | 0.5 | Gatterfosse |
| Sorbitol Instant® P300 | 5 | EM Industries |

b. Procedures:

1. Melt Gelucire and Pluronic, and dissolve ibuprofen into the mixture.
2. Add the above solution into Sorbitol Instant while stirring.
3. Cool down and pass through #20 mesh screen.

c. Dissolution Test Using the Dissolution Test as follows:

1. Measure 3.3 mg and 17.2 mg of ibuprofen (20 micron) and HydroSolve - Ibuprofen, respectively.
2. Measure the dissolution profiles using 700 ml of pH 7.2 buffer and paddle at 50 rpm.
3. Measure the absorbance at 221 nm.

| Results: | | |
|---|---|---|
| Time(min) | Ibuprofen | HydroSolve |
| 10 | 75.93 | 85.19 |
| 20 | 81.14 | 95.28 |
| 30 | 81.14 | 99.07 |

**Example 9:** Intrinsic Dissolution Profile of Phenytoin and HydroSolve- Phenytoin

a. Formulation #4:

| Excipient | Quantities (g) | Supplier |
|---|---|---|
| Phenytoin | I | Spectrum Quality Products |
| Gelucire® 50/13 | I | Gattefosse |
| Pluronic® F68 | I | BASF |

b. Procedures:

1. Melt Gelucire and Pluronic together.
2. Dissolve the phenytoin into above solution.
3. Congeal this suspension and pass through #20 mesh.
4. Measure about 32 and 110 mg of phenytoin and HydroSolve - phenytoin, respectively.
5. Measure the dissolution profiles using 900 ml of D1 water and Paddle Method at 50 rpm.

6. Using D1 water as blank, measure the absorbance at 220 nm of each sample which is filtered through 0.45 micron filters.

7. Calculate the percent released by standard curve:

$$Absorbance = 0.4072 \times Concentration \ (mg/100ml) + 0.0227$$

c. Results:

| Time (h) | % Dissolved (%) | |
|---|---|---|
| | Phenytoin | HydroSolve |
| 0.5 | 3.03 | 60.67 |
| 1 | 5.92 | 67.27 |
| 2 | 14.42 | 72 |
| 3 | 20.58 | 73.38 |

**Example 10**: The DSC Profiles for HydroSolve System of Phenytoin

1a. Formulation #1:

| Excipient | Quantities (g) | Supplier |
|---|---|---|
| Phenytoin | 1 | Spectrum Quality Products |
| Gelucire® 50/13 | 1 | Gatterfosse |
| Pluronic® F68 | 1 | BASF |
| Sorbitol Instant® P300 | 2.5 | EM Industries |

2b. Procedures:

1. Melt Gelucire and Pluronic together.
2. Disperse the phenytoin into above solution since pheytoin cannot completely.
3. Mix the above suspension with sorbitol P300 while stirring.
4. Measure the DSC profile.

2a. Formulation #4:

| Excipient | Quantities (g) | Supplier |
|---|---|---|
| Phenytoin | 1 | Spectrum Quality Products |
| Gelucire® 50/13 | 1 | Gatterfosse |
| Pluronic® F68 | 1 | BASF |

2b. Procedures:

1. Melt Gelucire and Pluronic together.
2. Dissolve/disperse the phenytoin into above system.
3. Congeal this suspension and pass through #20 mesh.
4. Measure the DSC profile.

c. Results:

| Material | PEAKS | |
|---|---|---|
| | Endotherm (°C) | Exotherm (°C) |
| Phenytoin: | 298.5 | |
| Pluronic® F68: | 56.6 | 163.4 |
| F#1: | 52.3 | 179.2 |
| F#4: | 52.4 | 174.2 |

The preceding examples can be repeated with similar success by substituting the generically or specifically described reactants and/or operating conditions of this invention for those used in the preceding examples.

**Claims**

1.  A pharmaceutical composition comprising a solid state solution or solid state dispersion of a poorly water soluble therapeutically active compound with an intrinsic water solubility of less than 10.0 g/l and a pharmaceutically acceptable excipient, said excipient comprising a mixture of:

    (a) polyglycolyzed glycerides composition wherein said composition is a mixture of monoesters, diesters and/ or triesters of glycerides of $C_{8-18}$-long-chain fatty acids and polyethylene glycol mono- and/or diesters of said fatty acids, and

    (b) polyoxypropylene-polyoxyethylene block copolymers,

    whereby the solubility of the poorly soluble therapeutically active compound in the pharmaceutical composition is enhanced and the mixture of the poorly soluble therapeutically active compound and the excipient is granulated, pelleted, extruded, extrusion spheronized, or spray congealed.

2.  A pharmaceutical composition of claim 1, wherein the composition of $C_{8-18}$-long-chain fatty acids in the glycerides in the composition of (a) comprise <10% $C_8$-fatty acid, <10% $C_{10}$-fatty acid, <50% $C_{12}$-fatty acid, <25% $C_{14}$-fatty acid, <50% $C_{16}$-fatty acid and <58% $C_{18}$-fatty acid.

3.  A pharmaceutical composition of claim 1 or 2, wherein (b) is a block copolymer of polyoxypropylene-polyoxyethylene having an average molecular weight from 3,000 to 15,000 and the ethoxylated portion of the block copolymer constitutes from 30 to 80% by weight of the molecule.

4.  A pharmaceutical composition of anyone of claims 1 to 3, wherein the composition comprising the solid state solution or solid state dispersion of a poorly water soluble therapeutically active compound and the excipient is in the form of a granule, particle, pellet, tablet or sphere and is coated with an agent that modifies the release profile of the therapeutically active compound.

5.  A pharmaceutical composition of claim 4, wherein the agent that modifies the release profile of the therapeutically active compound is a polymer of cellulose or a derivative thereof, alginic acid or a derivative thereof, polyvinyl alcohol or a derivative thereof acrylic acid polymer, polymethacrylates, acrylic acid or a derivative thereof, lactic acid or a derivative thereof or gelatin.

6.  A method for manufacturing an excipient for a pharmaceutical composition, said composition comprising a solid state solution or solid state dispersion of a poorly water soluble therapeutically active compound with an intrinsic water solubility of less than 10.0 g/l, said excipient comprising a mixture of:

    (a) polyglycolyzed glycerides composition and
    (b) polyoxypropylene-polyoxyethylene block copolymers,

    whereby the solubility of the poorly soluble therapeutically active compound in the pharmaceutical composition is enhanced, comprising:

heating said polyglycolyzed glycerides composition and polyoxypropylene-polyoxyethylene block co-polymer sufficiently to melt the ingredients, adding the therapeutic agent to the molten mixture of polyglycolyzed glycerides composition and polyoxypropylene-polyoxyethylene block co-polymer, maintaining the resulting mixture in the molten form, with constant stirring to ensure homogenous distribution of the drug in the system, and then subjecting the molten mixture to one or more of the following operations:

I) allowing the mixture to congeal to a solid mass, and then extruding the mixture through a hot melt extruder into a powder;

II) milling the mixture using equipment that would maintain the milling conditions at room temperature or below the melting point of the non-drug components of the system to enable milling the composition into a powder;

III) spray-congealing the mixture in a spray drier or fluidized bed drier to a powder;

IV) congealing the mixture onto one or more optional excipients in a spray drier, fluidized bed drier, rotor, high shear granulator, planetary mixer, blender, or any conventional food and pharmaceutical processing equipment;

V) formulating the powder obtained by one of the steps I to III in pharmaceutical tablets, capsules, powders for inhalation, suppositories, suspensions, and emulsions; or

VI) congealing the molten mixture onto a solid pharmaceutical tablet, capsule or granule.

7. A method of claim 6, further comprising adding one or more optional excipient, whereby:

(A) the solubility of the therapeutically-active compound in the polyglycolyzed glycerides composition : polyoxypropylene-polyoxyethylene block co-polymer mixture is increased, or

(B) the melting point of the non-drug components is set, whereby at least one melting point peak belonging to the non-drug components is present between 30-80° C in the final composition, when analyzed by thermal analytical techniques.

**Patentansprüche**

1. Pharmazeutische Zusammensetzung, enthaltend eine feste Lösung oder eine feste Dispersion einer wenig wasserlöslichen, therapeutisch wirksamen Verbindung mit einer intrinsischen Löslichkeit in Wasser von weniger als 10,0 g/l, und einen pharmazeutisch unbedenklichen Hilfsstoff, wobei der Hilfsstoff eine Mischung von

(a) einer polyglykolisierten Glyceridzusammensetzung, bei der es sich um eine Mischung von Monoestern, Diestern und/oder Triestern von Glyceriden von langkettigen $C_{8-18}$-Fettsäuren und Polyethylenglykolmono- und/oder -diestern dieser Fettsäuren handelt, und

(b) Polyoxypropylen-Polyoxyethylen-Blockcopolymeren

umfaßt, wobei die Löslichkeit der wenig löslichen, therapeutisch wirksamen Verbindung in der pharmazeutischen Zusammensetzung verbessert ist und die Mischung der wenig löslichen, therapeutisch wirksamen Verbindung und des Hilfsstoffes granuliert, pelletiert, extrudiert, extrusionssphäronisiert oder durch Sprühen zum Erstarren gebracht wird.

2. Pharmazeutische Zusammensetzung nach Anspruch 1, wobei die Zusammensetzung der langkettigen $C_{8-18}$-Fettsäuren in den Glyceriden in der Zusammensetzung von (a) <10% $C_8$-Fettsäure, <10% $C_{10}$-Fettsäure, <50% $C_{12}$-Fettsäure, <25% $C_{14}$-Fettsäure, <50% $C_{16}$-Fettsäure und <58% $C_{18}$-Fettsäure umfaßt.

3. Pharmazeutische Zusammensetzung nach Anspruch 1 oder 2, wobei es sich bei (b) um einen Blockcopolymer von Polyoxypropylen-Polyoxyethylen mit einem durchschnittlichen Molekulargewicht von 3000 bis 15 000 handelt und der ethoxylierte Anteil des Blockcopolymers 30 bis 80 Gew.-% des Moleküls ausmacht.

4. Pharmazeutische Zusammensetzung nach einem der Ansprüche 1 bis 3, wobei die Zusammensetzung die feste Lösung oder die feste Dispersion einer wenig wasserlöslichen, therapeutisch wirksamen Verbindung und den Hilfsstoff in Form von Granulat, Teilchen, Pellets, Tabletten oder Sphären umfasst und mit einem Mittel überzogen ist, das das Freisetzungsprofil der therapeutisch wirksamen Verbindung modifiziert.

**5.** Pharmazeutische Zusammensetzung nach Anspruch 4, wobei es sich bei dem Mittel, das das Freisetzungsprofil der therapeutisch wirksamen Verbindung modifiziert, um ein Polymer von Cellulose oder einem Derivat davon, Alginsäure oder einem Derivat davon, Polyvinylalkohol oder einem Derivat davon, Acrylsäurepolymer, Polymethacrylate, Acrylsäure oder einem Derivat davon, Milchsäure oder einem Derivat davon oder Gelatine handelt.

**6.** Verfahren zur Herstellung eines Hilfsstoffs für eine pharmazeutische Zusammensetzung, wobei die Zusammensetzung eine feste Lösung oder eine feste Dispersion einer wenig wasserlöslichen, therapeutisch wirksamen Verbindung mit einer intrinsischen Löslichkeit in Wasser von weniger als 10,0 g/l umfaßt, wobei der Hilfsstoff eine Mischung von

(a) einer polyglykolisierten Glyceridzusammen-Setzung und

(b) Polyoxypropylen-Polyoxyethylen-Blockcopolymeren

umfaßt, wobei die Löslichkeit der wenig löslichen, therapeutisch wirksamen Verbindung in der pharmazeutischen Zusammensetzung verbessert ist, bei dem man:

die polyglykolisierte Glyceridzusammensetzung und das Polyoxypropylen-Polyoxyethylen-Blockcopolymer soweit erhitzt, daß die Bestandteile schmelzen, der geschmolzenen Mischung der polyglykolisierten Glyceridzusammensetzung und des Polyoxypropylen-Polyoxyethylen-Blockcopolymers das therapeutische Mittel zusetzt, die so erhaltene Mischung im geschmolzenen Zustand hält, wobei, um sicherzustellen, daß das Arzneimittel homogen im System verteilt ist, ständig gerührt wird, und die geschmolzene Mischung dann einem oder mehreren der folgenden Behandlungsschritte unterzieht:

I) man läßt die Mischung zu einer festen Masse erstarren und extrudiert die Mischung dann aus einem Hotmelt-Extruder, so daß man ein Pulver erhält;

II) man mahlt die Mischung unter Einsatz von Gerät, mit dem die Mahlbedingungen bei Raumtemperatur bzw. unter dem Schmelzpunkt der Nicht-Arzneimittel-Komponenten des Systems aufrechterhalten werden können, so daß man die Zusammensetzung zu einem Pulver vermahlen kann;

III) man läßt die Mischung in einem Sprühtrockner oder einem Wirbelschichttrockner zu einem Pulver sprüherstarren;

IV) man läßt die Mischung in einem Sprühtrockner, einem Wirbelschichttrockner, einem Rotor, einem Granulator mit hohen Scherkräften, einem Planetenmischer, einem Mischer oder herkömmlichem Gerät zur Verarbeitung von Nahrungsmitteln und Pharmazeutika auf einem oder mehreren fakultativen Hilfsstoffen erstarren;

V) man formuliert das durch einen der Schritte I bis III erhaltene Pulver zu pharmazeutischen Tabletten, Kapseln, Inhalationspulvern, Zäpfchen, Suspensionen und Emulsionen; oder

VI) man läßt die geschmolzene Mischung auf einer festen pharmazeutischen Tablette oder Kapsel oder festem pharmazeutischem Granulat erstarren.

**7.** Verfahren nach Anspruch 6, bei dem man weiterhin einen oder mehrere fakultative Hilfsstoffe zusetzt, wobei:

(A) die Löslichkeit der therapeutisch wirksamen Verbindung in der polyglykolisierten Glyceridzusammensetzung/Polyoxypropylen-Polyoxyethylen-Blockcopolymer-Mischung erhöht wird, oder

(B) der Schmelzpunkt der Nicht-Arzneimittel-Komponenten eingestellt wird, wobei in der Endzusammensetzung wenigstens ein Schmelzpunktpeak der Nicht-Arzneimittel-Komponenten bei Analyse durch thermische Analyseverfahren zwischen 30 und 80°C liegt.

**Revendications**

**1.** Composition pharmaceutique comprenant une solution à l'état solide ou une dispersion à l'état solide d'un composé peu soluble dans l'eau, ayant une activité thérapeutique, dont la solubilité intrinsèque dans l'eau est inférieure à 10,0 g/l, et un excipient pharmaceutiquement acceptable, ledit excipient comprenant un mélange :

(a) d'une composition de glycérides polyglycolysés dans laquelle ladite composition est un mélange de monoesters, de diesters et/ou de triesters de glycérides d'acides gras à chaîne longue en $C_{8-18}$, et de monoesters et/ou diesters de polyéthylèneglycol desdits acides gras, et
(b) de copolymères séquencés polyoxypropylène-polyoxyéthylène,

grâce auquel la solubilité du composé peu soluble ayant une activité thérapeutique dans la composition pharmaceutique est renforcée et le mélange du composé peu soluble ayant une activité thérapeutique et de l'excipient est granulé, pastillé, extrudé, sphéronisé par extrusion ou congelé par atomisation.

2. Composition pharmaceutique selon la revendication 1, dans laquelle la composition d'acides gras à chaîne longue en $C_{8-18}$ dans les glycérides, dans la composition de (a), comprend <10% d'acide gras en $C_8$, <10% d'acide gras en $C_{10}$, <50% d'acide gras en $C_{12}$, <25% d'acide gras en $C_{14}$, <50% d'acide gras en $C_{16}$ et <58% d'acide gras en $C_{18}$.

3. Composition pharmaceutique selon la revendication 1 ou 2, dans laquelle (b) est un copolymère séquencé de polyoxypropylène-polyoxyéthylène ayant une masse moléculaire moyenne de 3 000 à 15 000 et la partie éthoxylée du copolymère séquencé constitue de 30 à 80% en poids de la molécule.

4. Composition pharmaceutique selon l'une quelconque des revendications 1 à 3, dans laquelle la composition comprend la solution à l'état solide ou la dispersion à l'état solide d'un composé peu soluble dans l'eau, ayant une activité thérapeutique, et l'excipient est sous forme d'un granulé, d'une particule, d'une pastille, d'un comprimé ou d'une sphère et est enrobé d'un agent qui modifie le profil de libération du composé ayant une activité thérapeutique.

5. Composition pharmaceutique selon la revendication 4, dans laquelle l'agent qui modifie le profil de libération du composé ayant une activité thérapeutique est un polymère de cellulose ou un de ses dérivés, de l'acide alginique ou un de ses dérivés, un poly alcool vinylique ou un de ses dérivés, un polymère d'acide acrylique, des polyméthacrylates, de l'acide acrylique ou un de ses dérivés, de l'acide lactique ou un de ses dérivés, ou de la gélatine.

6. Procédé de fabrication d'un excipient pour une composition pharmaceutique, ladite composition comprenant une solution à l'état solide ou une dispersion à l'état solide d'un composé peu soluble dans l'eau, ayant une activité thérapeutique, dont la solubilité intrinsèque dans l'eau est inférieure à 10,0 g/l, ledit excipient comprenant un mélange:

(a) d'une composition de glycérides polyglycolysés et
(b) de copolymères séquencés polyoxypropylène-polyoxyéthylène,

grâce auquel la solubilité du composé peu soluble ayant une activité thérapeutique dans la composition pharmaceutique est renforcée, comprenant les étapes consistant à:

chauffer suffisamment ladite composition de glycérides polyglycolysés et le copolymère séquencé polyoxypropylène-polyoxyéthylène pour faire fondre les ingrédients, ajouter l'agent thérapeutique au mélange fondu de composition de glycérides polyglycolysés et de copolymère séquencé polyoxypropylène-polyoxyéthylène, maintenir le mélange obtenu sous forme fondue, en agitant constamment pour assurer une répartition homogène du médicament dans le système, puis soumettre le mélange fondu à une ou plusieurs des opérations suivantes:

I) congeler le mélange jusqu'à l'obtention d'une masse solide, puis extruder le mélange à travers une extrudeuse à chaud pour obtenir une poudre;
II) broyer le mélange en utilisant un matériel qui maintiendra les conditions de broyage à la température ambiante ou au-dessous du point de fusion des constituants du système autres que le médicament pour permettre de broyer la composition afin d'obtenir une poudre,
III) congeler par atomisation le mélange dans un sécheur à atomisation ou un sécheur à lit fluidisé pour obtenir une poudre;
IV) congeler le mélange sur un ou plusieurs excipients facultatifs dans un sécheur à atomisation, un sécheur à lit fluidisé, un rotor, un granulateur à fort cisaillement, un mélangeur planétaire, un mixeur ou tout autre matériel classique de traitement des aliments et des produits pharmaceutiques;

V) formuler la poudre obtenue par une des étapes I à III en comprimés pharmaceutiques, gélules, poudres pour inhalation, suppositoires, suspensions et émulsions; ou

VI) congeler le mélange fondu sur un comprimé pharmaceutique, une gélule ou un granulé solide.

7. Procédé selon la revendication 6, comprenant en outre le fait d'ajouter un ou plusieurs excipients facultatifs, grâce auxquels:

(A) la solubilité du composé ayant une activité thérapeutique dans le mélange de composition de glycérides polyglycolysés et de copolymère séquencé polyoxypropyléne-polyoxyéthyléne est accrue, ou

(B) le point de fusion des constituants autres que le médicament est fixé, ce qui fait qu'au moins un pic de point de fusion appartenant aux constituants autres que le médicament est présent entre 30 et 80°C dans la composition finale, si on l'analyse par des techniques d'analyse thermique.